## Europäisches Patentamt

## European Patent Office

## Office européen des brevets

(19)

(11) **EP 0 804 165 B1**

(12) **EUROPÄISCHE PATENTSCHRIFT**

(45) Veröffentlichungstag und Bekanntmachung des Hinweises auf die Patenterteilung:
**17.10.2001 Patentblatt 2001/42**

(51) Int Cl.⁷: **A61K 9/14**, A61K 9/16, A61K 9/50

(21) Anmeldenummer: **96900575.0**

(22) Anmeldetag: **10.01.1996**

(86) Internationale Anmeldenummer:
**PCT/EP96/00073**

(87) Internationale Veröffentlichungsnummer:
**WO 96/21428 (18.07.1996 Gazette 1996/33)**

(54) **FESTE ARZNEIMITTELFORM MIT POLYMEREM MATERIAL VERTEILTEM WIRKSTOFF**

SOLID MEDICAMENT FORM WITH ACTIVE AGENT DISTRIBUTED IN POLYMER MATERIAL

FORME GALENIQUE SOLIDE A PRINCIPE ACTIF REPARTI DANS LE MATERIAU POLYMERE

(84) Benannte Vertragsstaaten:
**AT BE CH DE DK ES FR GB GR IE IT LI LU NL PT SE**
Benannte Erstreckungsstaaten:
**SI**

(30) Priorität: **14.01.1995 DE 19500977**

(43) Veröffentlichungstag der Anmeldung:
**05.11.1997 Patentblatt 1997/45**

(73) Patentinhaber: **LTS LOHMANN Therapie-Systeme AG**
**56626 Andernach (DE)**

(72) Erfinder:
- **ASMUSSEN, Bodo**
  **D-56710 Bendorf (DE)**
- **MÜLLER, Walter**
  **D-56565 Neuwied (DE)**
- **CREMER, Karsten**
  **D-53119 Bonn (DE)**

(74) Vertreter: **Flaccus, Rolf-Dieter, Dr.**
**Patentanwalt**
**Bussardweg 10**
**50389 Wesseling (DE)**

(56) Entgegenhaltungen:
**DE-A- 3 701 625      GB-A- 2 246 514**
**US-A- 4 151 273**

**Beschreibung**

Die Erfindung betrifft eine feste Arzneiform zur peroralen Applikation mit einem oder mehreren Wirkstoffen, welche in polymerem Material homogen verteilt ist.

Feste Wirkstoffeinbettungen in polymerem Material sind bereits seit längerem bekannt und werden in der pharmazeutischen Technologie zur Verfolgung unterschiedlicher Ziele eingesetzt. Eine Möglichkeit zur Systematisierung solcher Polymereinbettungen nach pharmazeutischen Gesichtspunkten ist die Einteilung in schnellfreisetzende und modifiziert bzw. retardiert freisetzende Arzneiformen.

Polymereinbettungen in schnellfreisetzenden Arzneiformen dienen in aller Regel der Beschleunigung der Wirkstoffauflösung in den Flüssigkeiten des Gastrointestinaltraktes. Die Auflösungsgeschwindigkeit eines festen Wirkstoffes ist nach Noyes und Whitney proportional zur wirksamen Feststoffoberfläche A und zur Differenz zwischen der Sättigungskonzentration $C_s$ und der tatsächlichen Wirkstoffkonzentration gemäß der Gleichung

$$\frac{dC}{dt} = k_1 \cdot A \cdot (C_s - C) \cdot V^{-1} \qquad (I)$$

wobei V für das Flüssigkeitsvolumen steht und die Konstante $k_1$ sich aus dem Diffusionskoeffizienten D des Wirkstoffs in der Flüssigkeit und der Dicke h der ruhenden Diffusionsschicht nach der Formel ergibt.

Eine naheliegende galenische Möglichkeit zur Erhöhung der Auflösegeschwindigkeit von Wirkstoffen liegt demnach in der Vergrößerung der Oberfläche A z. B. durch Mikronisieren. Eine wirksame Oberflächenvergrößerung nach der obigen Gleichung (I) ist bei schwer benetzbaren Wirkstoffen auch durch den Zusatz von Netzmitteln zu erreichen, da unbenetzte Oberflächen nicht in A eingehen.

Bei einer sehr geringen Löslichkeit $C_s$ des Wirkstoffes führen solche Maßnahmen allein jedoch nicht immer zum Ziel, da die Differenz ($C_s$-C) sehr klein ist und eine nur geringe Auflösegeschwindigkeit bewirkt. In vielen dieser Fälle läßt sich mit Einbettung der Wirkstoffe in Polymere als festen Lösungen eine deutliche Verbesserung erzielen.

Beispielsweise zeigt US 4,151,273 einen Weg auf, den schwerlöslichen Wirkstoff Griseofulvin als feste Lösung in Polyethylenglykol einzubetten, mit der nach peroraler Applikation eine schnellere Wirkstoffauflösung im Gastrointestinaltrakt und damit eine bessere Bioverfügbarkeit als bei herkömmlichen Zubereitungen erzielt werden kann. Weitere Beispiele für feste Wirkstofflösungen finden sich in J. Pharm. Sci., 54, S. 1145 - 1148 (1965).

Auch Wirkstoffeinbettungen in Form von festen Dispersionen in hydrophilen Polymeren können einen positiven Effekt auf die Wirkstoffauflösung haben.

Beispielsweise wird in US 4,933,360 eine schnellösliche feste Dispersion von Chlorthalidon in Polyvinylpyrrolidon beschrieben, die zu einer gegenüber handelsüblichen Zubereitungen deutlich verbesserten Wirkstoffaufnahme in Probanden geführt hat.

Umgekehrt läßt sich mit der Einbettung von Wirkstoffen in weniger gut wasserlöslichen Polymeren eine deutliche Verzögerung der Freisetzung bzw. Wirkstoffauflösung erreichen.

Diese Zubereitungen lassen sich einteilen in erodierbare und erosionsresistente Matrices. Unter den erodierbaren Matrices sind solche zu verstehen, welche sich im Laufe der Freisetzung entweder langsam auflösen oder einer anderen Art des Masseabbaus unterliegen. Dabei wird der Wirkstoff in der Regel vorwiegend an den Randzonen des Polymers freigesetzt. Dagegen bleiben erosionsresistente Matrices über den Freisetzungsverlauf weitgehend erhalten; ihre Löslichkeit ist sehr gering und die Kohärenz groß. Aus ihnen werden Wirkstoffe duch Diffusion freigesetzt, weshalb solche Zubereitungen auch Diffusionsmatrices genannt werden.

Für die Herstellung dieser Zubereitungen sind verschiedene Verfahren bekannt. Zu nennen sind unter anderem sog. Copräzipitate, Coevaporate, Coextrudate, Sprüh-, und Schmelzeinbettungen (Sucker et al., Pharmazeutische Technologie, Thieme Verlag 1991, S. 250f). Auch die weitere Verarbeitung solcher Einbettungen, falls sie nicht wie im Falle mancher Coextrudate direkt zu applizierbaren Arzneiformen führen, durch Zerkleinern zu Pulvern, Mischen mit weiteren Hilfsstoffen und anschließendes Tablettieren oder Abfüllen in Hartgelatinekapseln ist dem Fachmann geläufig (siehe z.B. US 4,933,360). Dabei haben die konventionellen Arzneiformen Tablette und Hartgelatinekapsel den Vorteil, daß hinsichtlich der Anforderungen zur Dosierungsgenauigkeit, Produktionsgeschwindigkeit und Wirtschaftlichkeit äußerst leistungsfähige Herstellungstechnologien zur Verfügung stehen.

Nachteile des Standes der Technik bestehen in erheblichen Aufwand bei der Zerkleinerung der Polymereinbettungen vor der Weiterverarbeitung zur endgültigen Arzneiform. Eine Zerkleinerung der Bulkware führt je nach Material, verwendeter Mühle und Mahlbedingungen zu verschieden kleinen und verschieden geformten Teilchen. Aus der Sicht moderner Anforderungen an die Reinheit von Arzneimitteln sind meist Mühlen wegen ihres Materialabriebes abzulehnen. Bei den aufwendigen Zerkleinerungsprozessen besteht die Gefahr der Veränderung des physikochemischen Zustandes, z. B. durch streßinitiierte thermodynamische Stabilisierung von ursprünglich glasartig amorphen Einbettungen durch Kristallisation oder bei festen Suspensionen sogar durch eine weitgehende Trennung von Wirkstoff und Polymer. Derart gravierenden Veränderungen kann durch die Wahl der Mahlbedingungen entgegengewirkt werden, wobei eine hohe Beanspruchung der Zubereitung zu vermeiden ist und der Zerkleinerungsgrad eher gering gehalten wird. Hierdurch gelangt man zu pelletartigen, sphärisch bis unregelmäßig geformten Partikeln, welche allerdings den Nachteil besitzen, daß das Verhältnis ihrer räumlichen Ausdehnung zur Oberfläche die Wirkstofffreisetzung erheblich beeinträchtigen kann. So ist es erklärbar, daß sich die Freisetzungsgeschwindigkeit eines Wirkstoffes aus pelletartigen Partikeln eines retardierenden Polymers, in welches er eingebettet ist, nach einer relativ raschen Initialfreisetzung des in der Nähe der Partikeloberfläche lokalisierten Wirkstoffes durch die kontinuierlich wachsenden Diffusionsstrecken im Polymer ständig verringert. Eine Umgehung dieses Effektes der wachsenden Diffusionsstrecken ist nach dem gegenwärtigen Stand der Technik nur durch feinste Mahlung der Einbettung möglich, welche die angegebenen Nachteile und Gefahren mit sich bringt.

Dieser Erfindung liegt die Aufgabe zugrunde, Wirkstoffeinbettungen in polymerem Material in einer solchen Form in Arzneizubereitungen einzusetzen, die nicht mit den aufgeführten Nachteilen der im Stand der Technik bekannten Pellets und Pulver behaftet ist.

Die Lösung der Aufgabe gelingt durch die Schaffung einer festen Arzneiform, welche eine homogene Verteilung von Wirkstoff in polymerem Material in Form von flachen Bruchstücken enthält. Auf diese Weise ist es möglich, unter Umgehung einer Feinmahlung die Diffusionsstrecken zur Polymeroberfläche besonders kurz und damit relativ konstant zu halten.

Flache Bruchstücke im Sinne dieser Erfindung sind Stücke oder Abschnitte mit schollen-, plättchen- bzw. blattartiger Form, wie sie z.B. beim Zerbrechen von filmartigem Material entstehen. Sie besitzen eine gegenüber ihrer Längen- und Breitenausdehnung wesentlich geringere Dicke, von 1 µm bis 500 µm, wobei dünne Filme bzw. Folien bevorzugt sind.

Eine Möglichkeit, solche flachen Partikel zu erzeugen, besteht im Auswalzen sphärisch oder unregelmäßig geformter Teilchen, wie Sie aus schonenden Zerkleinerungsprozessen gewonnen werden können, zwischen zwei gegensinnig rotierenden Walzen. Voraussetzung dieser Methode ist eine ausreichende plastische Verformbarkeit des Einbettungsmaterials; eine zu hohe Elastizität würde nicht zur dauerhaften Abflachung der Teilchen führen, während sprödes Material unter diesen Bedingungen in kleinere, unregelmäßige Bruchstücke zerbricht.

Wesentlich vorteilhafter und bevorzugt ist die Erzeugung von flachen Partikeln aus flächigem, filmartigen Material. Für die Herstellung eines solchen filmartigen Materials stehen leistungsfähige Beschichtungstechniken zur Verfügung, welche auch bereits in der pharmazeutischen Industrie - beispielsweise zur Fertigung transdermaler therapeutischer Systeme - eingesetzt werden. Auf diese Weise kann auch sprödes Einbettungsmaterial erzeugt und weiterverarbeitet werden. Mit dieser Methode lassen sich erfindungsgemäß sowohl feste Lösungen als auch feste Dispersionen herstellen.

Alternativ können Polymerfilme mit homogen verteiltem Wirkstoff auch durch Extrusionsverfahren hergestellt werden. Die Extrusion mittels einer Förderapparatur und einer temperierbaren Schlitzdüse und anschließendes Strecken wird in der technischen Folienproduktion breit verwendet.

Häufig ist es bei der Formulierung von Einbettungen angezeigt, dem polymeren Material außer dem Wirkstoff noch Hilfsstoffe zuzugeben. Dazu zählen z.B. Tenside, Weichmacher, Kristallisationsverzögerer, Antioxidantien, Säuren, Basen usw.

Die Grobzerkleinerung von filmartigem Material zu flachen Bruchstücken läßt sich schonend mit Hilfe von weitgehend abriebfreien Rund- und Walzenbrechern sowie mit Schneidemaschinen bewerkstelligen. Diese Maschinen lassen sich in der Regel so einstellen, daß sie zu Teilchengrößen (Längenausdehnung) von 0,2 bis 2 mm führen, welches die bevorzugte Teilchengröße für das Einbettungsprodukt ist. Die Beanspruchung des Materials ist dabei wesentlich geringer als bei einer Feinmahlung oder gar Mikronisierung.

Die erfindungsgemäß bevorzugten Arzneiformen sind Tabletten und Hartgelatinekapseln, da sie gut applizierbar und von Patienten weitgehend akzeptiert sind, ebenso weil für sie Fertigunstechnologien zur Verfügung stehen, die allen heutigen Anforderungen hinsichtlich der Beachtung der "GMP"-Richtlinien (Good Manufacturing Practices) für pharmazeutische Hersteller, insbesondere der Dosierungsgenauigkeit, aber auch der wirtschaftlich effizienten Fertigung großer Stückzahlen entsprechen.

Hierzu kann das polymere Material mit eingebettetem Wirkstoff und gegebenenfalls Hilfsstoffen mit weiteren Hilfsstoffen vermischt werden. Dazu zählen z. B. physiologisch unbedenkliche Füllstoffe, Bindemittel, Fließregulierungs-, Schmier- und Trennmittel, Antioxidantien, Farbstoffe bzw. Pigmente, Aromen, Netzmittel, Hydrophilisierungsmittel, Hydrophobisierungsmittel, Löslichkeitsverbesserer, Zerfallsmodulatoren, Substanzen zur Einstellung des pH-Wertes usw. Die Mischung kann anschließend auf herkömmlichen Maschinen tablettiert oder in Hartgelatinekapseln abgefüllt werden.

In eine erfindungsgemäße Arzneiform lassen sich auch mehrere Wirkstoffe einarbeiten, soweit dies erforderlich erscheint. Das kann auf unterschiedliche Weise geschehen. Einerseits ist es möglich, im polymeren Material selbst mehr als einen Wirkstoff einzubetten. Andererseits können auch mehrere Einbettungen in einer Arzneiform vereinigt werden. Schließlich ist es möglich, eingebettete Wirkstoffe neben nichteingebetteten in derselben Arzneiform vorliegen zu haben. Somit ergibt sich eine Vielzahl von galenischen Möglichkeiten zur Steuerung der Freisetzungsgeschwindigkeit der einzelnen Wirkstoffe auch unabhängig voneinander.

Die neue Arzneiform bietet einen zusätzlichen Vorteil, indem sie es ermöglicht, auf wirkungsvolle Weise mucoadhäsive Hilfsstoffe therapeutisch einzusetzen. Wird der Wirkstoff nämlich in einem polymeren Material mit mucoadhäsiven Eigenschaften oder unter Zusatz von mucoadhäsiven Hilfsstoffen eingebettet und in eine schnellzerfallenden Tablette oder Kapsel eingebracht, so werden nach dem Primärzerfall der Arzneiform im Gastrointestinaltrakt eine Vielzahl flacher Partikel mit besonders großer Gesamtkontaktfläche zur Mucosa und einer dadurch gegenüber anderen Zubereitungen wesentlich verbesserten Mucoadhäsivität freigesetzt. Dabei ist eine Verstärkung aller positiven Effekte der Mucoadhäsion, wie z.B. die Verlängerung der Verweildauer in den oberen Abschnitten des Gastrointestinaltraktes sowie die aufgrund der kurzen Diffusionsstrecke erhöhte Absorptionsrate höhermolekularer Wirkstoffe erreichbar (Lenaerts et al., Bioadhesive Drug Delivery Systems, CRC Press 1990).

Die Freisetzung der Wirkstoffe aus den Einbettungen der bekannten Polymere wird durch deren Form als flache Bruchstücke wesentlich gefördert bzw. leicht steuerbar, d.h. wahlweise beschleunigt oder verzögert. In formstabil und unzersetzt bleibenden polymeren Materialien ist die Diffusion der Wirkstoffe, auch durch die Wahl der Foliendicke, beschleunigt und einstellbar. In polymeren Materialien, die durch chemische Struktur und das Molekulargewicht den Wirkstoff in der Körperflüssigkeit durch Lösen, Zersetzung der Polymeren, Erosion oder Quellung freisetzen, ergibt die erfindungsgemäße flache Form der Polymerbruchstücke galenisch stark erweiterte Möglichkeiten.

**Patentansprüche**

1. Feste Arzneiform zur peroralen Applikation von Wirkstoffen, die eine homogene Wirkstoffverteilung in polymerem Material enthält, **dadurch gekennzeichnet, daß** das wirkstoffhaltige polymere Material in Form flacher Bruchstücke mit einer gegenüber der Längen- und Breitenausdehnung geringen Dicke von 1-500 μm vorliegt.

2. Feste Arzneiform nach Anspruch 1, **dadurch gekennzeichnet, daß** das Wirkstoff enthaltende, polymere Material in Form flacher Bruchstücke direkt oder nach Mischung mit zur Tablettierung geeigneten Hilfsstoffen zu einer Tablette gepreßt ist.

3. Feste Arzneiform nach Anspruch 1, **dadurch gekennzeichnet, daß** das Wirkstoff enthaltende, polymere Material in Form flacher Bruchstücke direkt oder nach Mischung mit weiteren pharmazeutisch geeigneten Hilfsstoffen als Kapselfüllmaterial verwendet wird.

4. Feste Arzneiform nach einem der Ansprüche 1 - 3, **dadurch gekennzeichnet, daß** sie mehrere Wirkstoffe enthält.

5. Feste Arzneiform nach einem der Ansprüche 1 - 4, **dadurch gekennzeichnet, daß** die Wirkstoffe im polymeren Material gelöst vorliegen.

6. Feste Arzneiform nach einem der Ansprüche 1 bis 4, **dadurch gekennzeichnet, daß** der oder die Wirkstoffe im polymeren Material dispergiert vorliegen.

7. Feste Arzneiform nach einem der Ansprüche 1 - 6, **dadurch gekennzeichnet, daß** das polymere Material zusätzliche Hilfsstoffe wie Tenside, Weichmacher, Stabilisatoren, Kristallisationsverzögerer, Antioxidantien, Dochtsubstanzen oder Substanzen zur Einstellung des pH-Wertes enthält.

8. Feste Arzneiform nach einem der Ansprüche 1 - 7, **dadurch gekennzeichnet, daß** das polymere Material in Form eines dünnen Films vorliegt.

9. Feste Arzneiform nach einem der Ansprüche 1 - 8, **dadurch gekennzeichnet, daß** das polymere Material mucoadhäsive Eigenschaften besitzt.

10. Feste Arzneiform nach einem der Ansprüche 1 - 9, **dadurch gekennzeichnet, daß** das polymere Material im Freisetzungsmedium mit oder ohne Erhalt seiner chemischen Struktur erodierbar oder löslich ist.

11. Feste Arzneiform nach einem der Ansprüche 1 - 10, **dadurch gekennzeichnet, daß** das polymere Material im Freisetzungsmedium quellbar ist.

12. Feste Arzneiform nach einem der Ansprüche 1 - 11, **dadurch gekennzeichnet, daß** das polymere Material im Freisetzungsmedium formstabil bleibt.

13. Feste Arzneiform nach einem der Ansprüche 11 oder 12, **dadurch gekennzeichnet, daß** der Wirkstoff überwiegend durch Diffusion freigesetzt wird.

**Claims**

1. Solid drug form for peroral administration of active compounds which comprises a homogeneous distribution of active compound in polymeric material, **characterised in that** the active compound-containing polymeric material is present in the form of flat fragments which have a small thickness of 1 - 500 μm in comparison with their lengthwise and widthwise extensions.

2. Solid drug form according to Claim 1, **characterized in that** the polymeric material comprising active compound is pressed in the form of flat fragments to a tablet, directly or after mixing with auxiliaries suitable for tablet-making.

**3.** Solid drug form according to Claim 1, **characterized in that** the polymeric material comprising active compound is used in the form of flat fragments as a capsule filling material directly or after mixing with further pharmaceutically suitable auxiliaries.

**4.** Solid drug form according to one of Claims 1 - 3, **characterized in that** it comprises more than one active compound.

**5.** Solid drug form according to one of Claims 1 - 4, **characterized in that** the active compounds are present in the polymeric material as a solution.

**6.** Solid drug form according to one of Claims 1 to 4, **characterized in that** the active compound or compounds are present in the polymeric material in the form of a dispersion.

**7.** Solid drug form according to one of Claims 1 - 6, **characterized in that** the polymeric material comprises additional auxiliaries, such as surfactants, plasticizers, stabilizers, crystallization retardants, antioxidants, wick substances or substances for adjusting the pH.

**8.** Solid drug form according to one of Claims 1 - 7, **characterized in that** the polymeric material is in the form of a thin film.

**9.** Solid drug form according to one of Claims 1 - 8, **characterized in that** the polymeric material has mucoadhesive properties.

**10.** Solid drug form according to one of Claims 1 - 9, **characterized in that** the polymeric material is erodable or soluble in the release medium with or without retaining its chemical structure.

**11.** Solid drug form according to one of Claims 1 - 10, **characterized in that** the polymeric material is swellable in the release medium.

**12.** Solid drug form according to one of Claims 1 - 11, **characterized in that** the polymeric material remains dimensionally stable in the release medium.

**13.** Solid drug form according to one of Claims 11 or 12, **characterized in that** the active compound is chiefly released by diffusion.

**Revendications**

**1.** Forme médicamenteuse solide pour l'application de substances actives par voie orale, qui contient une distribution homogène de substance active dans une matière polymère, **caractérisée en ce que** la matière polymère contenant les substances actives est présente sous la forme de fractions plates et possédant une épaisseur minime, par rapport à la longueur et par rapport à la largeur, de 1 à 500 μm.

**2.** Forme médicamenteuse solide selon la revendication 1, **caractérisée en ce que** la matière polymère contenant la substance active, sous la forme de fractions plates, est transformée en un comprimé directement ou après mélange avec des adjuvants appropriés pour la transformation en comprimés.

**3.** Forme médicamenteuse solide selon la revendication 1, **caractérisée en ce que** la matière polymère contenant la substance active, sous la forme de fractions plates est utilisée directement ou après mélange avec d'autres adjuvants pharmaceutiquement appropriés, sous la forme d'une matière d'encapsulage.

**4.** Forme médicamenteuse solide selon l'une quelconque des revendications 1 à 3, **caractérisée en ce qu'**elle contient plusieurs substances actives.

**5.** Forme médicamenteuse solide selon l'une quelconque des revendications 1 à 4, **caractérisée en ce que** les substances actives sont présentes sous forme dissoute dans la matière polymère.

**6.** Forme médicamenteuse solide selon l'une quelconque des revendications 1 à 4, **caractérisée en ce que** la ou les substances actives sont présentes sous forme dispersée dans la matière polymère.

**7.** Forme médicamenteuse solide selon l'une quelconque des revendications 1 à 6, **caractérisée en ce que** la matière polymère contient des adjuvants supplémentaires tels que des agents tensioactifs, des plastifiants, des stabilisateurs, des ralentisseurs de la cristallisation, des antioxydants, des substances à effet de mèche ou des substances pour régler la valeur du pH.

**8.** Forme médicamenteuse solide selon l'une quelconque des revendications 1 à 7, **caractérisée en ce que** la matière polymère est présente sous la forme d'un film mince.

**9.** Forme médicamenteuse solide selon l'une quelconque des revendications 1 à 8, **caractérisée en ce que** la matière polymère possède des propriétés mucoadhésives.

**10.** Forme médicamenteuse solide selon l'une quelconque des revendications 1 à 9, **caractérisée en ce que** la matière polymère peut être érodée ou est soluble dans le milieu de libération, en conservant ou non sa structure chimique.

**11.** Forme médicamenteuse solide selon l'une quelconque des revendications 1 à 10, **caractérisée en ce que** la matière polymère peut gonfler dans le milieu de libération.

**12.** Forme médicamenteuse solide selon l'une quelconque des revendications 1 à 11, **caractérisée en ce que** la matière polymère manifeste une stabilité dimensionnelle dans le milieu de libération.

**13.** Forme médicamenteuse solide selon l'une quelconque des revendications 11 ou 12, **caractérisée en ce que** la substance active est libérée principalement par diffusion.